(19) Europäisches Patentamt
European Patent Office
Office européen des brevets

(11) Veröffentlichungsnummer: **0 028 755**
**B1**

(12) **EUROPÄISCHE PATENTSCHRIFT**

(45) Veröffentlichungstag der Patentschrift: **16.01.91**

(51) Int. Cl.$^5$: **A 01 N 43/54,** C 07 D 239/26

(21) Anmeldenummer: **80106574.9**

(22) Anmeldetag: **25.10.80**

# (54) Mittel zur Regulierung des Pflanzenwachstums, deren Herstellung und deren Verwendung.

(30) Priorität: **07.11.79 DE 2944850**

(43) Veröffentlichungstag der Anmeldung:
**20.05.81 Patentblatt 81/20**

(45) Bekanntmachung des Hinweises auf die Patenterteilung:
**16.01.91 Patentblatt 91/03**

(84) Benannte Vertragsstaaten:
**AT BE CH DE FR GB IT LI NL**

(56) Entgegenhaltungen:
**EP-A-0 001 399**
**US-A-3 869 456**

(73) Patentinhaber: **BAYER AG**
**D-5090 Leverkusen 1 Bayerwerk (DE)**

(72) Erfinder: **Holmwood, Graham, Dr.**
**Katernberger Strasse 184**
**D-5600 Wuppertal 1 (DE)**
Erfinder: **Lürssen, Klaus, Dr.**
**A.-Kierspel-Strasse 89**
**D-5070 Bergisch-Gladbach (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 028 755 B1

**Beschreibung**

Die vorliegende Erfindung betrifft die Verwendung von bekannten Pyrimidin-butanol-Derivaten als Wirkstoffe zur Regulierung des Pflanzenwachstums.

Es ist bereits bekannt geworden, daß bestimmte Pyrimidin-butanol-Derivate fungizide Eigenschaften besitzen (vgl. DE—OS 27 42 173).

Weiterhin ist bereits bekannt geworden, daß bestimmte Pyrimidin-Derivate, wie beispielsweise α-(4-Fluorphenyl)-α-phenyl-5-pyrimidinmethanol und α-(2-Chlorphenyl)-α-(4-fluorphenyl)-5-pyrimidinmethanol unter anderem auch wachstumsregulierende Eigenschaften aufweisen (vergleiche die US-Patentschriften 3 818 009 und 3 869 456). Allerdings ist die Wirksamkeit dieser Stoffe, vor allem bei niedrigen Aufwandmengen, nicht immer ganz befriedigend.

Es wurde gefunden, daß die bekannten Pyrimidin-butanol-Derivate der Formel

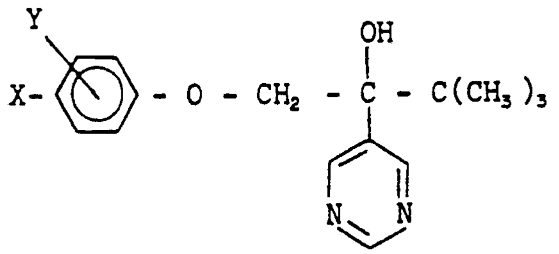

(I)

in welcher

X für Wasserstoff, Halogen, Alkyl, Alkoxy oder gegebenenfalls durch Halogen substituiertes Benzyloxy steht und

Y für Wasserstoff oder Halogen steht,

starke wachstrumsregulierende Eigenschaften aufweisen.

Die Verbindung der Formel (I) besitzen ein asymmetrisches Kohlenstoffatom; sie können deshalb in den beiden optischen Isomeren oder als Racemat vorliegen.

Überraschenderweise zeigen die erfindungsgemäß verwendbaren Pyrimidin-butanol-Derivate der Formel (I) eine bessere pflanzenwachstumsregulierende Wirkung als die bekannten Pyrimidin-Derivate α-(4-Fluorphenyul)-α-phenyl-5-pyrimidinmethanol und α-(2-Chlorphenyl)-α-(4-fluorphenyl)-5-pyrimidinmethanol, welches chemisch und wirkungsmäßig naheliegende Verbinbungen sind. Die erfindungsgemäße Verwendung der Pyrimidin-butanol-Derivate stellt somit eine Bereicherung der Technik dar.

Die erfindungsgemäß verwendbaren Pyrimidin-butanol-Derivate sind durch die Formel (I) allgemein definiert. In dieser Formel steht *X* vorzugsweise für Wasserstoff, Fluor, Chlor, Brom, Alkyl mit 1 bis 4 Kohlenstoffatomen, Alkoxy mit 1 bis 4 Kohlenstoffatomen oder gegebenenfalls durch Chlor substituiertes Benzyloxy. *Y* steht vorzugsweise für Wasserstoff, Fluor, Chlor oder Brom.

Ganz besonders bevorzugt sind diejenigen Verbindungen der Formel (I), in denen *X* für Wasserstoff, Fluor, Chlor, Methyl, Ethyl, Isopropyl, Methoxy, Ethoxy, Isopropoxy, Benzyloxy oder Chlorbenzyloxy steht; und *Y* für Wasserstoff, Chlor oder Fluor steht.

Die erfindungsgemäß verwendbaren Pyrimidin-butanol-Derivate sind bekannt (vergleiche DE—OS 27 42 173). Sie lassen sich herstellen, indem man Phenoxymethyl-tert.-butylketone der Formel

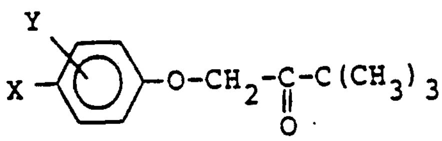

(II)

in welcher

X und y die oben angegebene Bedeutung haben,

mit 5-Halogen-pyrimidin der Formel

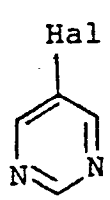

(III)

in welcher

Hal für Halogen steht,

in Gegenwart eines inerten organischen Lösungsmitttels oder Lösungsmittelgemisches, wie

beispielsweise Diethylether und Tetrahydrofuran, und in Gegenwart einer alkali-metall-organischen Verbindung, wie beispielsweise n-Butyllithium, als Base bei Temperaturen zwischen −150°C und −50°C unter Inertgas, wie beispielsweise Stickstoff, umsetzt. Zur Isolierung der Endprodukte wird das Reaktionsgemisch in üblicher Weise aufgearbeitet und gereinigt; gegebenenfalls wird ein Säureadditionssalz oder ein Metallsalz-Komplex hergestellt.

Die erfindungsgemäß verwendbaren Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträgte bezogen auf die Bodenfläche erzielt werden können Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragsteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, sodaß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzebau auch in Verbindung mit einer Wuchshemmung sehr erwünscht sein kann. Andererseits ist es aber auch möglich, das Wachstums der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann eine vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der

Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor odder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüberhinaus kann mit Wachstumsregulatoren auch eine zeitliche Konzentrierung der Reife erzielt werden. Damit werden die Voraussetzungen dafür geschaffen, daß z.B. Tabak, Tomaten oder Kaffee eine vollständige mechanische oder mauelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigung durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Besonders hervorzuheben ist, daß die erfindungsgemäßen Verbindungen auch eine Wuchshemmung bei Reis zeigen.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglykol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol- Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder der daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die Aufwandmengen können in einem größeren Bereich variiert werden. Im allgemeinen verwendet man pro Hektar Bodenfläche 0,01 bis 50 kg, bevorzugt 0,05 bis 10 kg an Wirkstoff

Für Aufwandmengen können in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung der Wachstumsregulatoren in einem bevorzugten Zeitraum vorgenommen wird, dessen genaue Abgrenzungen sich nach den klimatischen Gegebenheiten richtet.

In entsprechenden Aufwandmengen zeigen die erfindungsgemäß verwendbaren Wirkstoffe auch eine herbizide Wirksamkeit.

Im folgenden wird die wachstumsregulierende Wirksamkeit der erfindungsgemäß verwendbaren Pyrimidin-butanol-Derivate durch einige Beispiele illustriert.

## Beispiel A

Hemmung des Seitentriebwachstums bei Tabak

Lösungsmittel: 30 Gewichtsteile Dimethylformamid
Emulgator: 1 Gewichtsteil Polyoxyethylen-Sorbinatar-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Tabakpflanzen werden in Gewächshaus bis zur Entfaltung des 7. Folgeblattes angezogen. In diesem Stadium wird die apikale Vegetationsspitze entfernt und die Pflanzen werden tropfnass mit den Wirkstoffzubereitungen besprüht. Nach 3 Wochen werden die Seitentriebe der Pflanze herausgebrochen und gewogen. Das Gewicht der Seitentriebe der behandelten Pflanzen wird mit dem der Kontrollpflanzen verglichen. 100% Hemmung bedeuten das Fehlen von Seitentrieben und 0% ein Seitentriebwachstum entsprechend dem der Kontrollpflanzen.

Wirkstoffe, Wirkstoffkonzentrationen und Resultate gehen aus der nachfolgenden Tabelle hervor.

.belle A

Hemmung des Seitentriebwachstums bei Tabak

| Wirkstoff | Konzentration in % | Wuchshemmung in % |
|---|---|---|
| 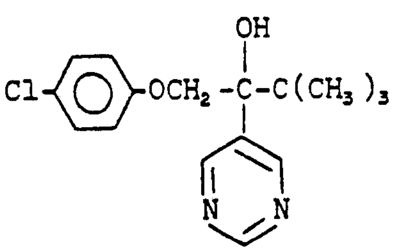 (1) | 0,2 | 76 |
| - (Kontrolle) | - | 0 |

## Beispiel B

Wuchshemmung bei Sojabohnen

Lösungsmittel: 30 Gewichtsteile Dimethylformamid
Emulgator: 1 Gewichsteil Polyoxyethylen-Sorbinat-Monolaurat

Zur Herstellung einer zweckmäßigen Wirkstoffzubereitung vermischt man 1 Gewichtsteil Wirkstoff mit

den angegebenen Mengen Lösungsmittel und Emulgator und füllt mit Wasser auf die gewünschte Konzentration auf.

Sojabohnenpflanzen werden in Gewächshaus bis zur vollen Entfaltung des ersten Folgeblattes angezogen. In diesem Stadium werden die Pflanzen tropfnaß mit den Wirkstoffzubereitungen besprüht. Nach 3 Wochen wird bei allen Pflanzen des Zuwachs gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Wirkstoffe, Wirkstoffkonzentrationen und Resultate gehen aus der nachfolgenden Tabelle hervor.

Tabelle B

Wuchshemmung bei Sojabohnen

| Wirkstoffe | Wirkstoffkonzentration in % | Wuchshemmung in % |
|---|---|---|
| 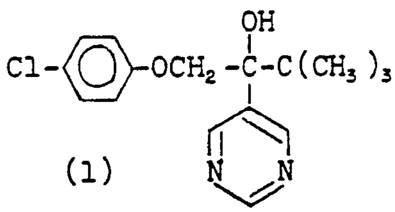 (1) | 0,05 | 100 |
| 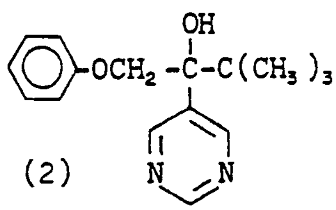 (2) | 0,05 | 80* |
| 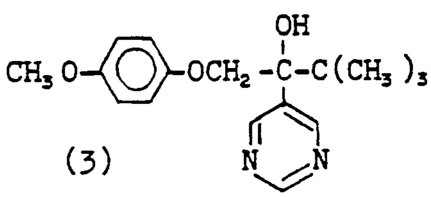 (3) | 0,05 | 50 |
| 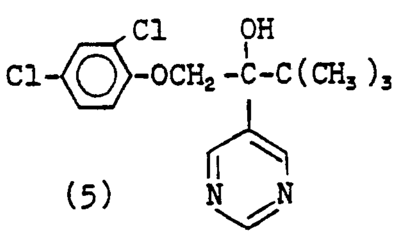 (5) | 0,05 | 85* |

Tabelle B (Fortsetzung)

Wuchshemmung bei Sojabohnen

| Wirkstoffe | Wirkstoffkonzentration in % | Wuchshemmung in % |
|---|---|---|
| 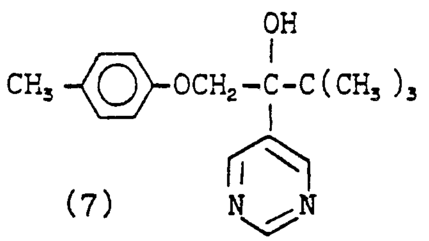 (7) | 0,05 | 70* ** |
| 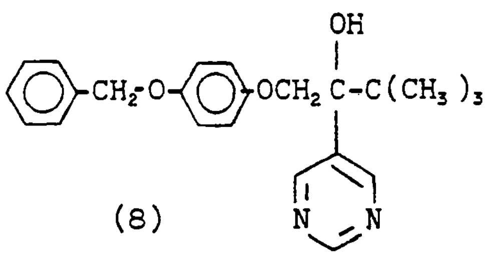 (8) | 0,05 | 30* |
| - (Kontrolle) | - | 0 |

* = verstärkte Seitentriebbildung
** = dunkelgrüne Blattfärbung

Beispiel C

Wuchshemmung bei Gehölzen (Acer)

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Einjährige Sämlinge mit einer Wuchshöhe von 25 cm werden mit den Wirkstoffzubereitungen tropfnass besprüht. Nach 6 Wochen Wachstum im Gewächshaus wird der Zuwachs gemessen und die Wuchshemmung in Prozent des Zuwachses der Kontrollpflanzen berechnet. 100% Wuchshemmung bedeuten den Stillstand des Wachstums und 0% ein Wachstum entsprechend dem der Kontrollpflanzen.

Wirkstoffe, Wirkstoffkonzentrationen und Resultate gehen aus der nachfolgenden Tabelle hervor.

**Tabelle: C**

Wuchshemmung bei Gehölzen (Acer)

| Wirkstoff | Konzentration in % | Wuchshemmung in % |
|---|---|---|
| 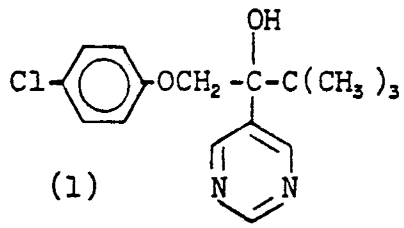 (1) | 0,2 | 91 |
| - (Kontrolle) | - | 0 |

Beispiel D

Wuchshemmung bei Gehölzen (Alnus)

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Einjährige Sämlinge mit einer Wuchshöhe von 25 cm werden mit den Wirkstoffzubereitungen tropfnaß besprüht. Nach 6 Wochen Wachstum im Gewächshaus wird der Zuwachs gemessen und die Wuchshemmung in Prozent des Zuwachses der Kontrollpflanzen berechnet. 100% Wuchshemmung bedeuten den Stillstand des Wachstums und 0% ein Wachstum entsprechend dem der Kontrollpflanzen.

Wirkstoffe, Wirkstoffkonzentrationen und Resultate gehen aus der nachfolgenden Tabelle hervor.

**Tabelle: D** (Alnus)

Wuchshemmung bei Gehölzen

| Wirkstoff | Konzentration in % | Wuchshemmung in % |
|---|---|---|
| 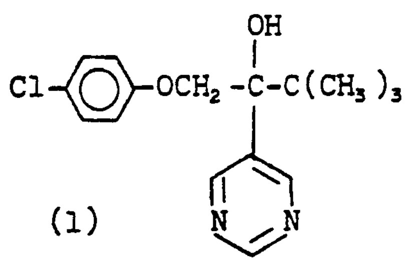 (1) | 0,2<br>0,4 | 77<br>84 |
| - (Kontrolle) | - | 0 |

Beispiel E

Wuchshemmung bei Gras (Festuca pratensis)

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit

den angegebenen Mengen Lösungsmittel und Emulgator und füllt mit Wasser auf die gewünschte Konzentration auf.

Gras (Festuca pratensis) wird im Gewächshaus bis zu einer Wuchshöhe von 5 cm angezogen. In diesem Stadium werden die Pflanzen mit den Wirkstoffzubereitungen tropfnaß besprüht. Nach 3 Wochen wird der Zuwachs gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Wirkstoffe, Wirkstoffkonzentrationen und Resultate gehen aus der nachfolgenden Tabelle hervor.

Tabelle E

Wuchshemmung bei Gras (Festuca pratensis)

| Wirkstoff | Konzentration in % | Wuchshemmung in % |
|---|---|---|
| 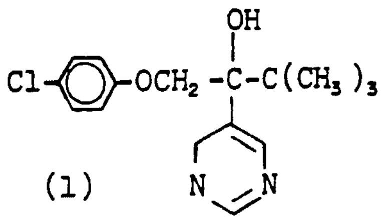 (1) | 0,05 | 90 |
| 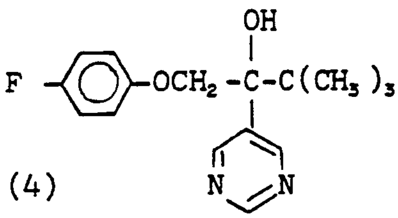 (4) | 0,05 | 70 |
| 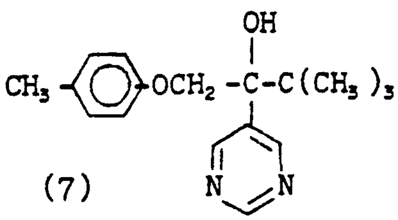 (7) | 0,05 | 55 |
| - (Kontrolle) | - | 0 |
| 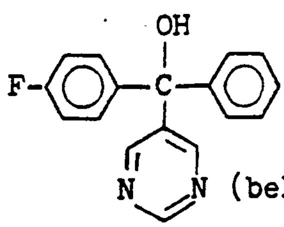 (bekannt) | 0,05 | 0 |
| 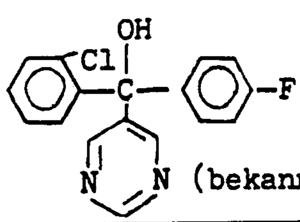 (bekannt) | 0,05 | 0 |

Beispiel F

Wuchshemmung bei Gerste

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gerstenpflanzen werden im Gewächshaus bis zum 2-Blatt-stadium angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100% Wuchshemmung den Stillstand des Wachstums und 0% ein Wachstum entsprechend der Kontrollpflanzen.

Wirkstoffe, Wirkstoffkonzentrationen und Resultate gehen aus der nachfolgenden Tabelle hervor.

Tabelle: F

Wuchshemmung bei Gerste

| Wirkstoff | Konzentration in % | Wuchshemmung in % |
|---|---|---|
| 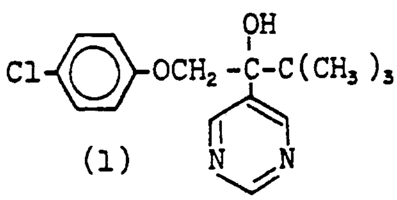 (1) | 0,050<br>0,025 | 42<br>24 |
| 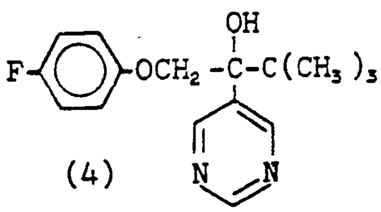 (4) | 0,050 | 35 |
| 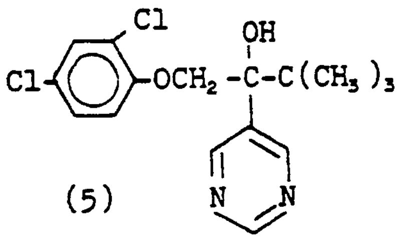 (5) | 0,050 | 25 |
| 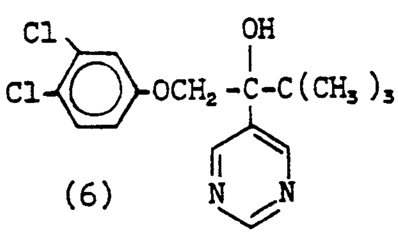 (6) | 0,050 | 50 |
| 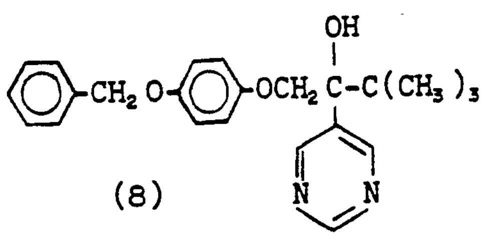 (8) | 0,050 | 75 |

Tabelle: F (Fortsetzung)

Wuchshemmung bei Gerste

| Wirkstoff | Konzentration in % | Wuchshemmung in % |
|---|---|---|
| - (Kontrolle) | - | 0 |
| 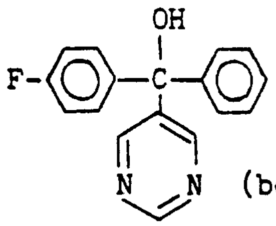 (bekannt) | 0,05 | 0 |
| 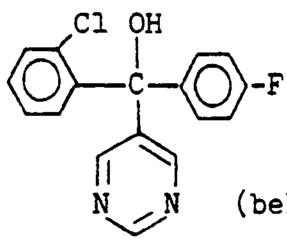 (bekannt) | 0,05 | 0 |

Beispiel G

Wuchsbeeinflussung bei Zuckerrüben

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnaß mit den Wirkstoffzubereitung besprüht. Nach 14 Tagen wird der Zuwachs der Pflanzen gemessen und die Wuchsbeeinflussung in Prozent des Zuwachses der Kontrollpflanzen berechnet. Es bedeuten 0% Wuchsbeeinflussung ein Wachstum entsprechend dem der Kontrollpflanzen. Negative Werte kennzeichnen eine Wuchshemmung, positive Werte eine Wuchsförderung gegenüber den Kontrollpflanzen.

Wirkstoffe, Wirkstoffkonzentrationen und Resultate gehen aus der nachfolgenden Tabelle hervor.

Tabelle G

Wuchsbeeinflussung bei Zuckerrüben

| W i r k s t o f f | Konzentration in % | Wuchshemmung in % |
|---|---|---|
| 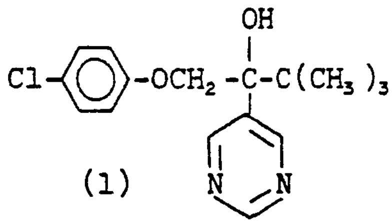 (1) | 0,05 | -90*** |
| 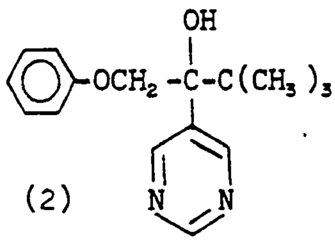 (2) | 0,05 | -65*** |
| 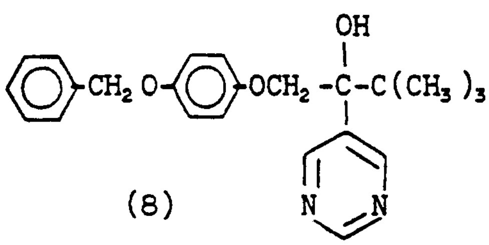 (8) | 0,05 | -30*** |
| 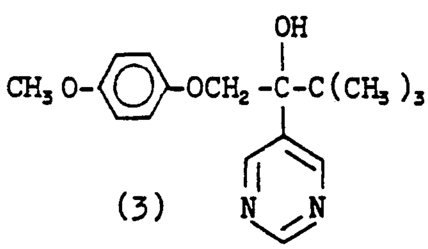 (3) | 0,05 | -75 |
| 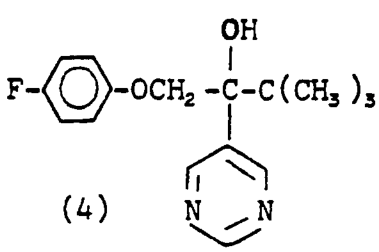 (4) | 0,05 | -75*** |

Tabelle G (Fortsetzung)

Wuchsbeeinflussung bei Zuckerrüben

| Wirkstoff | Konzentration in % | Wuchshemmung in % |
|---|---|---|
| 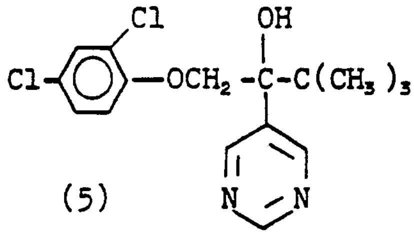 (5) | 0,05 | -80** |
| 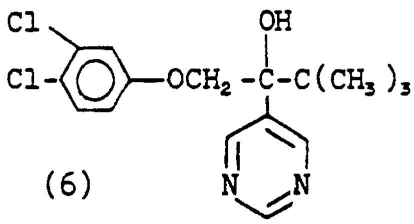 (6) | 0,05 | -75** |
| 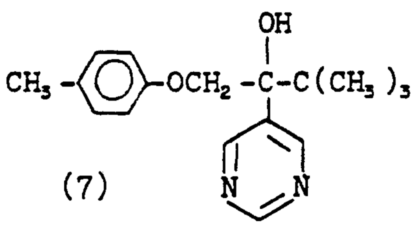 (7) | 0,05 | -75 |
| 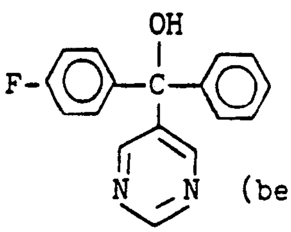 (bekannt) | 0,05 | -10** |
| 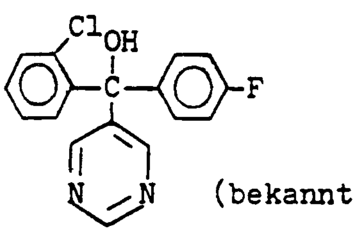 (bekannt) | 0,05 | -30** |
| (Kontrolle) | - | = 0 |

* = dunkelgrüne Blätter

** = besonders dicke Blätter

Beispiel H

Wuchshemmung bei Reis

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Reispflanzen werden im Gewächshaus in 10 × 10 × 10 cm Töpfen in Erde bis zum 2-Blattstadium angezogen. In diesem Stadium werden die Pflanzen tropfnaß mit den Wirkstoffzubereitungen besprüht. Nach 10 Tagen wird bei allen Pflanzen der Zuwachs gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Weitere Versuchsdaten und die Resultate dieses Versuches gehen aus der nachfolgenden Tabelle hervor.

Tabelle H

Wuchshemmung bei Reis

| Wirkstoff | Konzentration in % | Wuchshemmung in % |
|---|---|---|
| 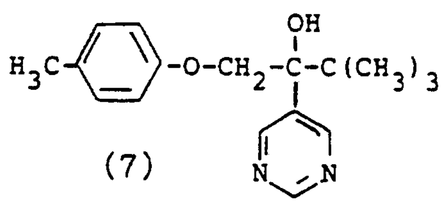 (7) | 0,0125 | 9 |
| | 0,0250 | 35 |
| | 0,0500 | 57 |
| 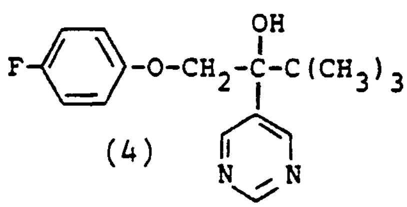 (4) | 0,0125 | 26 |
| 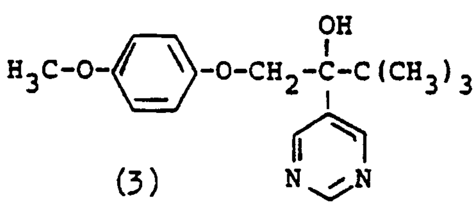 (3) | 0,0125 | 10 |
| 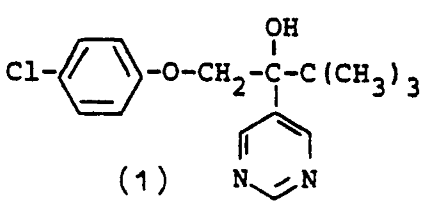 (1) | 0,0125 | 4 |
| | 0,0250 | 17 |

Tabelle H (Fortsetzung)

Wuchshemmung bei Reis

| Wirkstoff | Konzentration in % | Wuchshemmung in % |
|---|---|---|
| 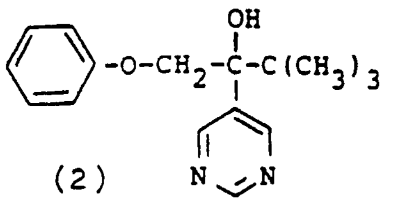 (2) | 0,0125 | 17 |
| 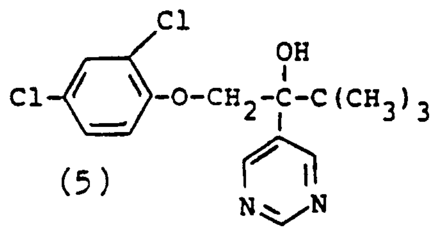 (5) | 0,0125 | 17 |
| Kontrolle | - | = 0 |

Beispiel I

Wuchshemmung bei Wasser-Reis var. Nihonbare

5 Teile Wirkstoff werden mit 2,5 Teilen Newkalgen CP-50, 30 Teilen Bentonit und 62,5 Teilen Talkum in einem Mixer vermischt. 20 Teile Wasser werden zugesetzt. Der Brei wird durch Löcher von 0,5 mm Durchmesser gepreßt und getrocknet. Hieraus resultiert ein Graulat mit einer Korngröße von 0,5 mm Ø und etwa 0,7 mm Länge.

10 Tage alte Pflanzen werden in 25 × 20 × 10 cm große Schalen gepflanzt, in denen Wasser über der Erde angestaut ist. Nach 10 Tagen wird die Wirkstoffzubereitung ins Wasser appliziert. Nach weiteren 14 Tagen wird die Wuchshöhe der Pflanzen gemessen.

In diesem Test zeigen die Verbindungen aus den Beispielen 2, 4, 5, 7 und 3 eine sehr gute wuchshemmende Wirksamkeit.

Herstellungsbeispiele

Beispiel 1

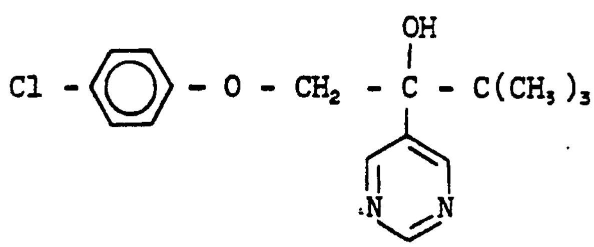

Eine Lösung von 22,65 (0,1 Mol) 1-(4-Chlorphenoxy)-3,3-dimethyl-butan-2-on in 110 ml absolutem Tetrahydrofuran und 70 ml absolutem Ether wird unter trockener Stickstoffatmosphäre auf −120°C abgekühlt. Dazu tropft man eine Lösung von 15,9 g (0,1 Mol) 5-Brompyrimidin in 50 ml absolutem Tetrahydrofuran. Anschließend werden bei −120°C 50 ml einer 15%-igen Lösung von n-Butyllithium in n-Hexan langsam zugetropft. Man läßt zunächst 2 Stunden bei einer Temperatur von ca. −110°C, danach über Nacht bei −78°C nachrühren. Das Reaktionsgemisch wird auf Raumtemperatur erwärmt, mit 100 ml 10%-iger Ammonium-chlorid-Lösung und 200 ml Essigester versetzt und die wässrige Phase abgetrennt. Die organische Phase wird nacheinander einmal mit 1 n Salzsäure und zweimal mit gesättigter Natriumchloridlösung gewaschen, über Natriumsulfat getrocknet und eingeengt. Der Rückstand wird mit Ether aufgeschlämmt, der Feststoff abgeseugt und aus Acetonitril umkristallisiert. Man erhält 12,3 g (50% der Theorie bezogen auf n-Butyllithium) 1-(4-Chlorphenoxy)-3,3-dimethyl-2-(pyrimidin-5-yl)-butan-2-ol vom Schmelzpunkt 172—174°C.

Analog werden die in der folgenden Tabelle 1 aufgeführten Verbindungen der Formel

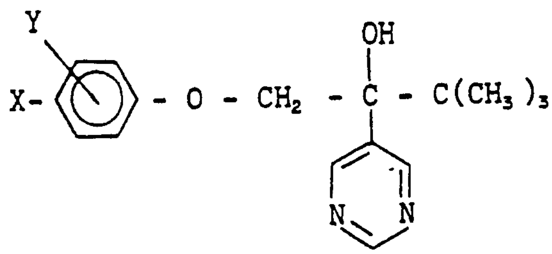

(I)

erhalten:

Tabelle 1

| Bsp.Nr. | X | Y | Schmelzpunkt(°C) |
|---|---|---|---|
| 2 | H | H | 127-29 |
| 3 | $CH_3O$- | H | 136-37 |
| 4 | F | H | 163,5-64,5 |
| 5 | Cl | 2-Cl | 96-99 |
| 6 | Cl | 3-Cl | 155-57 |
| 7 | $CH_3$ | H | 152-53,5 |
| 8 | 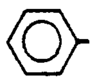-$CH_2$-O- | H | 122-24 |

**Patentansprüche**

1. Verwendung von Pyrimidin-butanol-Derivaten der Formel

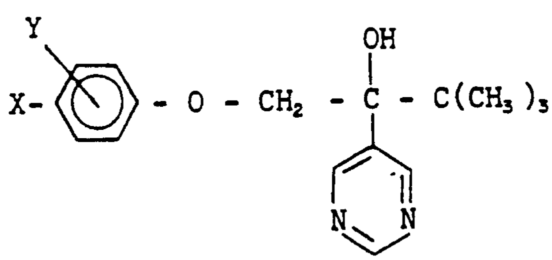

(I)

in welcher

X für Wasserstoff, Halogen, Alkyl, Alkoxy oder gegebenenfalls durch Halogen substituiertes Benzyloxy steht undd

Y für Wasserstoff oder Halogen steht,

zur Regulierung des Pflanzenwachstums.

2. Verwendung des Pyrimidin-butanol-Derivates der Formel

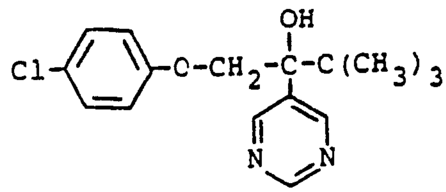

zur Regulierung des Pflanzenwachstums.

3. Verwendung des Pyrimidin-butanol-Derivates der Formel

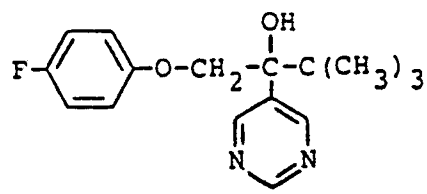

zur Regulierung des Pflanzenwachstums.

4. Verwendung des Pyrimidin-butanol-Derivates der Formel

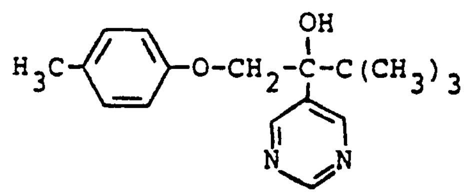

zur Regulierung des Pflanzenwachstums.

## Revendications

1. Utilisation de dérivés de pyrimidine-butanol de formule

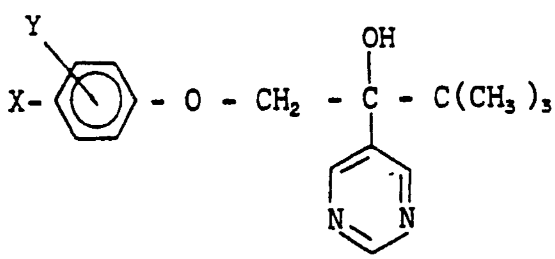

(I)

dans laquelle

X représente l'hydrogène, un halogène, un groupe alkyle, un groupe alcoxy ou éventuellement un groupe benzyloxy substitué par un halogène et

Y représente l'hydrogène ou un halogène comme régulateurs de croissance des plantes.

2. Utilisation du dérivé de pyrimidine-butanol de formule

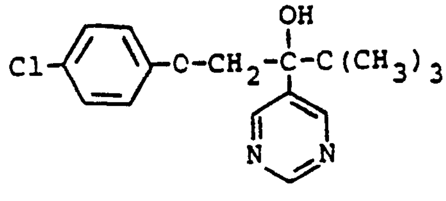

comme régulateur de croissance des plantes.

3. Utilisation du dérivé de pyrimidine-butanol de formule

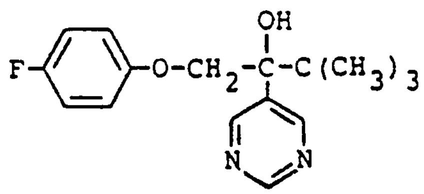

comme régulateur de croissance des plantes.

4. Utilisation du dérivé de pyrimidine-butanol de formule

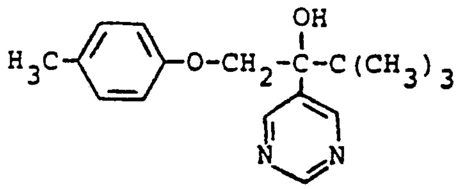

comme régulateur de croissance des plantes.

## Claims

1. Use of pyrimidine-butanol derivatives of the formula

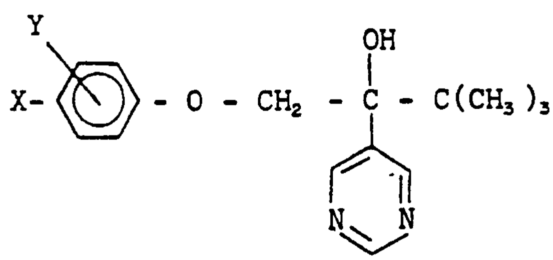

(I)

in which

X represents hydrogen, halogen, alkyl, alkoxy or benzyloxy which is optionally substituted by halogen and

Y represents hydrogen or halogen,

for regulating plant growth.

2. Use of the pyrimidine-butanol derivative of the formula

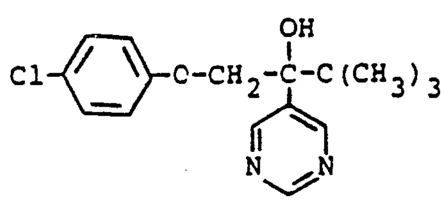

for regulating plant growth.

3. Use of the pyrimidine-butanol derivative of the formula

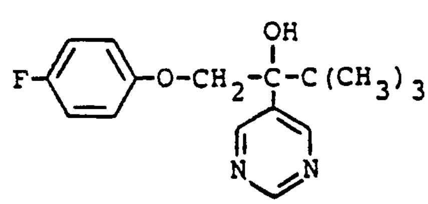

for regulating plant growth.

4. Use of the pyrimidine-butanol derivative of the formula

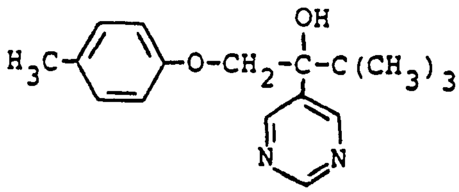

for regulating plant growth.